# EUROPEAN PATENT SPECIFICATION

(11) **EP 1 109 973 B1**
(45) Date of publication and mention of the grant of the patent: **05.07.2006**
(21) Application number: 00942226.2
(22) Date of filing: 29.06.2000
(51) Int. Cl.: E02D 5/18, E02D 17/13

(54) **Under-reamed diaphragm wall**
Schlitzwand mit erweitertem, geräumten Unterteil
Barrette moulée sous alésée

(30) Priority: 02.07.1999 GB 9915576
(43) Date of publication of application: 27.06.2001
(73) Proprietor: Cementation Foundations Skanska Limited, Rickmansworth, Hertfordshire WD3 9AS (GB)
(72) Inventor: SHOTTON, Peter, Gilbert, Flackwell Heath, Bucks HP10 9NA (GB); PAYNE, Ron, Iver, Bucks SL0 9QT (GB)
(74) Representative: Harman, Michael Godfrey
(86) International application number: PCT/GB2000/002507
(87) International publication number: WO 2001/002651

(56) References cited:
- DE-A- 2 008 575
- GB-A- 1 446 276
- JP-A- 59 145 822
- US-A- 4 290 652

## Description

This invention relates to the stabilisation of soil structures and, more particularly but not exclusively, to methods of stabilisation and tools for use in those methods.

It is known to construct an underground wall by excavating a trench and then filling the trench with reinforced concrete to form the wall. It is often desirable to stabilize the soil surrounding the wall, by reducing the chance of subsidence and achieving anchorage of the wall with the soil.

A known method of achieving this is to provide the wall with an enlargement running along its length below ground level. One proposal showing this is Riepel, DE 2 008 575, which shows an underground wall with an enlargement extending along the whole length of the wall. (In fact, it shows two such enlargements, one above the other and both above the base of the wall.) Another similar proposal is Kajima Kensetsu, JP 59-145822, which shows a more conventional structure comprising an underground wall with an enlargement at its base. The enlargement may protrude to only one side of the wall, or may protrude symmetrically on both sides of the wall.

To form such walls, it is necessary to excavate the trench and then form an enlarged space extending along it, before the concrete is poured to form the wall. In the case of Riepel, the enlarged spaces have, in section, the form of a circular sector of somewhat less than 60° with its vertex upwards. In the case of Kajima Kensetsu, the enlargement has (again in vertical section) the form of a flat base with, rising from it, sides which are initially vertical and then bent or angled to converge gradually to the wall width. The enlargement spaces are evidently formed by means of a vertical drill which has means at its lower end expandable to form the enlargement. (This is similar to the well known technique of forming a pile with an enlargement at its base.) The enlargement is evidently extended along the trench either by repeatedly lowering the drill at closely spaced intervals along the trench, or by moving the drill transversely along the trench.

The general object of the present invention is to provide an improved method and apparatus for constructing an underground wall with an enlargement extending along it below ground level.

Accordingly the invention provides a method of stabilizing soil by constructing a diaphragm wall the lower end of which is anchored in an under-reamed cavity filled with concrete, the method comprising constructing a trench and then under-reaming a cavity therein by lowering into the trench a tool having excavating means operable to engage the earth to be excavated and continuously rotatable to excavate the soil so engaged with, characterized in that said continuous excavating rotation is about an axis generally parallel to the bottom of the trench.

The invention also provides a tool for under-reaming a cavity in a trench for a diaphragm wall constructed according to the above method, characterized in that the tool comprises a cutting tool continuously rotatable about an axis generally parallel to the bottom of the trench to excavate the soil so engaged with in the direction of said axis and mounted on an arm pivotally swingable about said axis to expand the region excavated to a width greater than the width of the trench.

The tool preferably comprises a positioning arm; a supporting arm pivotally attached to a first point which is at or close to one end of said positioning arm; a cutting tool carried by said supporting arm and pivotally mounted thereon at a second point which is at or close to that end of said supporting arm remote from said positioning arm; a plate fixed in position relative to said supporting arm or integral therewith, the plate lying in or parallel to the plane swept out by said supporting arm as it rotates around said first point; and means for moving said plate so as to cause the supporting arm to pivot about said first point, thereby permitting controlled movement of said circular cutting tool in arcuate path.

Generally, the positioning arm will be an elongate member so as to facilitate use of the under-reaming tool at depths of several metres, or several tens of metres, below ground level. The length of the positioning arm and its form of construction will be selected according to the operating conditions in which the tool is required to function.

The cutting tool is advantageously circular in form.

The means for moving said plate so as to cause the supporting arm to pivot about said first point is preferably a rigid or semi-rigid linkage extending between (a) a point of connection on the plate itself and (b) a position close to the axis of the positioning arm and on that side of said first point opposite to said second pivot point when the supporting arm is positioned so as to extend as a continuation of the positioning arm. Thus if the positioning arm extends downwardly into the ground, one end of the linkage will be connected to the plate and the other end of the linkage will be connected to a point above the distal (bottom) end of the positioning arm. A suitable linkage can be provided by using a hydraulic ram.

Preferably, the plate is triangular, and more preferably it is in the form of an equilateral triangle.

The pivot point between the supporting arm and the positioning arm - termed herein the "first point" - may pass through the body of said plate. Conveniently said first point can be at or close to the centroid of the triangle when the plate is triangular.

The arrangements described permit the tool to be operated so as to generate an under-reamed cavity which is circular in cross-section in the plane of the cutting tool (which will often correspond to a vertical plane). Other forms of articulation between the cutting tool and the positioning arm may be adopted if desired; these may permit variations in the geometry of the cavity which is formed by use of the tool.

In one embodiment, the supporting arm is fixed in length; in other embodiments, its length can be varied. This variation in length also permits different cavity geometries to be formed if desired (either during a single under-reaming operation or so as to generate cavities of different dimensions in successive operations, whether at the same or different locations).

When a tool in accordance with this invention is used, the positioning arm will be located as desired, for example at the base of a trench which may typically be from 3 to 50 metres below ground level. The positioning arm is preferably located by means of a guide - e.g. a tubular structure through which the positioning arm and other components of the tool are passed.

For a better understanding of the invention, and to show how the same may be carried into effect, reference will now be made, by way of example, to the accompanying drawings, in which:

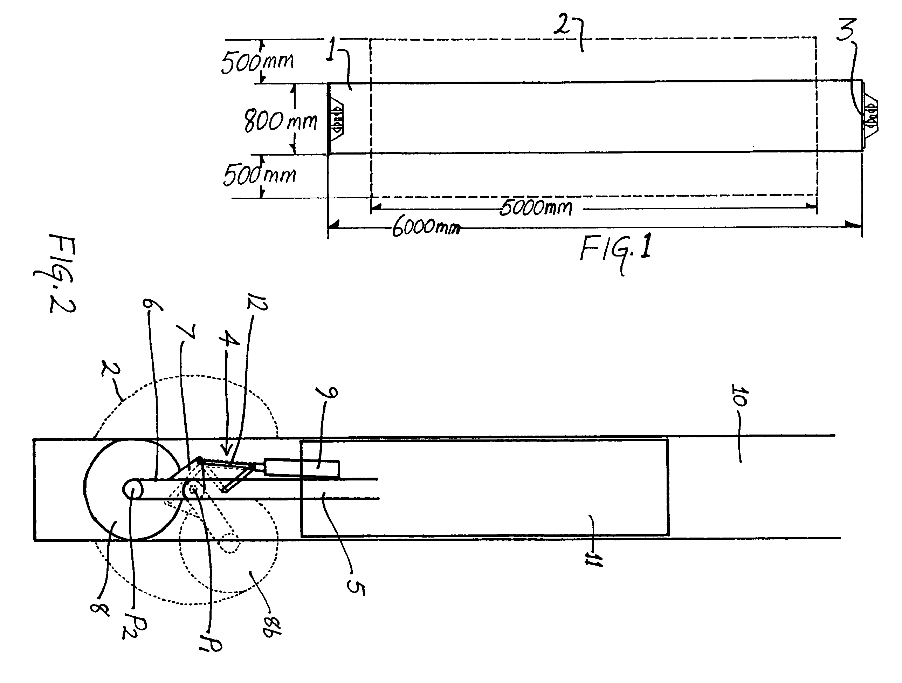

FIGURE 1 is a plan view of a trench in which a diaphragm wall of this invention is installed; and
FIGURE 2 is a side elevation view through the trench of Fig. 1 during construction with an under-reaming tool of this invention in position within the trench.

Referring to the drawings, a diaphragm wall 1 six metres in width is anchored, at its base, by a barrel-shaped anchorage 2 whose lateral extent exceeds that of wall 1 by 500mm on either side. The width of the anchorage is five metres - there being a section of wall approximately 500mm in extent adjacent to the stop ends 3 where the anchorage is absent.

In Fig. 2, an under-reaming tool 4 is shown at the base of trench 10. A tubular guide 11 is used to position the tool 4 within the trench. Tool 4 comprises an elongate positioning arm 5; a supporting arm 6; a rigid triangular plate 7; a circular cutting tool 8; and a hydraulic ram 9. Supporting arm 6 and plate 7 are fixed together; they are pivotally connected to the lower end of arm 5 at point P1, as shown. Cutting tool 8 is mounted on supporting arm 6 so as to be able to rotate about point P2. Hydraulic ram 9 connects with one of the triangular vertices of plate 7 by means of a rigid link 12.

When the tool is in use, hydraulic ram 9 is operated so as to cause arm 6, and hence cutting tool 8, to pivot about point P1. This pivotal movement moves the tool 8 with respect to the axis of arm 5; one position of tool 8 close to its extreme limit of movement is marked as 8b. The cutting tool 8 is hydraulically operated. The pivotal movement just described results in the formation of a cavity whose cross-section is generally circular. If the arm 5 is lifted upwardly after an initial cutting operation, the cavity will become barrel-shaped; if the lifting continues, the cavity will tend towards a slab-like form.

Operation of the cutting tool 8 is carried out in a conventional manner, e.g. in the presence of a drilling fluid and with extraction of debris through the guide member 11.

Concrete can be supplied to the cavity through a tremie (not shown). If desired, the concrete may be reinforced by conventional reinforcements - e.g. by steel rods or meshes.

## Claims

1. A method of stabilizing soil by constructing a diaphragm wall the lower end of which is anchored in an under-reamed cavity filled with concrete, the method comprising constructing a trench (10) and then under-reaming a cavity (2) therein by lowering into the trench a tool having excavating means operable to engage the earth to be excavated and continuously rotatable to excavate the soil so engaged with, **characterized in that** said continuous excavating rotation is about an axis generally parallel to the bottom of the trench.

2. A tool for under-reaming a cavity in a trench for a diaphragm wall constructed according to the method of claim 1, **characterized in that** the tool comprises a cutting tool (8) continuously rotatable about an axis generally parallel to the bottom of the trench to excavate the soil so engaged with in the direction of said axis and mounted on an arm (6) pivotally swingable about said axis to expand the region excavated to a width greater than the width of the trench.

3. A tool as claimed in claim 2, **characterized in that** the tool comprises a positioning arm (5); a supporting arm (6) pivotally attached to a first point (P1) which is at or close to one end of said positioning arm; a cutting tool (8) carried by said supporting arm and pivotally mounted thereon at a second point (P2) which is at or close to that end of the supporting arm remote from said positioning arm; a plate (7) fixed in position relative to said supporting arm or integral therewith, the plate lying in or parallel to the plane swept out by said supporting arm as it rotates around said first point; and means (9) for moving said plate so as to cause the supporting arm to pivot about said first point, thereby permitting controlled movement of said circular cutting tool in an arcuate path.

4. A tool as claimed in claim 3, **characterized in that** said positioning arm is an elongate member.

5. A tool as claimed in claim 3 or 4, **characterized in that** said cutting tool is circular in form.

6. A tool as claimed in claim 3, 4, or 5, **characterized in that** said means (9) for moving said plate so as to cause the supporting arm to pivot about said first point is a rigid or semi-rigid linkage (12) extending between (a) a point of connection on the plate itself and (b) a position close to the axis of the positioning arm and on that side of said first point opposite to said second pivot point when the supporting arm is positioned so as to extend as a continuation of the positioning arm.

7. A tool as claimed in claim 6, **characterized in that** said linkage is provided by a hydraulic ram.

8. A tool as claimed in any one of claims 3-7, **characterized in that** said plate is triangular.

9. A tool as claimed in claim 8, wherein said plate is in the form of an equilateral triangle.

10. A tool as claimed in any one of claims 3-9, **characterized in that** said first point (P1) passes through the body of said plate.

11. A tool as claimed in claims 8 and 10, **characterized in that** said first point is at or close to the centroid of the triangle when the plate is triangular.

12. A tool as claimed in any one of claims 3-11, **characterized in that** said supporting arm (6) is fixed in length.

13. A tool as claimed in any one of claims 3-11, **characterized in that** said supporting arm (6) is variable in length.

## Patentansprüche

1. Methode zur Stabilisierung des Erdreichs durch Einbau einer Schlitzwand, an deren unterem Ende sich ein geräumter, mit Beton gefüllter Hohlraum befindet, wobei die Methode das Ausheben eines Grabens (10) und danach die Herstellung eines Hohlraums (2) an diesem durch Unterräumen umfasst, indem ein Werkzeug mit Aushubmitteln in den Graben abgesenkt wird, die so betätigt werden können, dass sie das auszuhebende Erdreich erfassen und selber in ständiger Umdrehung gehalten werden können und das so erfasste Erdreich ausgehoben werden kann, **dadurch gekennzeichnet, dass** die besagte, kontinuierliche Aushebebewegung um eine Achse erfolgt, die im wesentlichen parallel zur Sohle des Grabens verläuft.

2. Werkzeug zum Herstellen eines Hohlraums in einem Graben durch Unterräumen für eine nach der Methode in Anspruch 1 einzubauende Schlitzwand, **dadurch gekennzeichnet, dass** es sich bei dem Werkzeug um ein kontinuierlich um eine im wesentlichen parallel zur Sohle des Grabens verlaufende Achse drehbares Schneidwerkzeug (8) zum Aushub des so gefassten Erdreichs handelt, das so in Richtung der besagten Achse eingreift und auf einem Arm (6) um die besagte Achse schwenkbar so montiert ist, dass der ausgehobene Bereich auf eine Breite erweitert werden kann, die größer als die Breite des Grabens ist.

3. Werkzeug gemäß Anspruch 2 **dadurch gekennzeichnet, dass** das Werkzeug einen Einstellarm (5); einen Tragarm (6), der schwenkbar an einem ersten Punkt (P1) angelenkt ist, der sich an oder in dichter Nähe eines Endes des besagten Einstellarms befindet; ein von dem besagten Tragarm getragenes Schneidwerkzeug (8), welches schwenkbar auf diesem an einem zweiten Punkt (P2) montiert ist, der sich an oder in dichter Nähe des von dem Einstellarm entfernt liegenden Endes des besagten Tragarms befindet; eine in einer relativen Lage zu dem besagten Tragarm befestigte oder mit diesem integrierend ausgeführte Platte (7), die in oder parallel zu der Ebene verläuft, die von dem besagten Tragarm bestrichen wird, während sich dieser um den besagten ersten Punkt dreht; und Mittel (9) zur Bewegung der besagten Platte aufweist, die eine Schwenkbewegung des Tragarms um den besagten ersten Punkt bewirken, so dass eine gesteuerte Bewegung des besagten kreisförmigen Schneidwerkzeugs über einen bogenförmigen Pfad ermöglicht wird.

4. Werkzeug gemäß Anspruch 3, **dadurch gekennzeichnet, dass** der besagte Einstellarm ein längliches Glied ist.

5. Werkzeug gemäß Anspruch 3 oder 4, **dadurch gekennzeichnet, dass** das besagte Schneidwerkzeug kreisförmig ist.

6. Werkzeug gemäß Anspruch 3, 4 oder 5, **dadurch gekennzeichnet, dass** das besagte Mittel (9) zur Bewegung der besagten Platte derartig, dass der Tragarm um den besagten ersten Punkt verschwenkt wird, vorzugsweise als starres oder halbstarres Gestänge (12) ausgeführt ist, das zwischen (a) einem Verbindungspunkt mit der eigentlichen Platte und (b) einer Position dicht in der Nähe an der Achse des Einstellarms und auf der Seite des besagten ersten Punkts gegenüber dem besagten zweiten Drehpunkt verläuft, wenn der Tragarm so eingestellt ist, dass er als Verlängerung des Einstellarms wirkt.

7. Werkzeug gemäß Anspruch 6, **dadurch gekennzeichnet, dass** das besagte Gestänge mit einem Hydraulikkolben ausgeführt sein kann.

8. Werkzeug gemäß einem der Ansprüche 3 bis 7, **dadurch gekennzeichnet, dass** die besagte Platte dreieckig ist.

9. Werkzeug gemäß Anspruch 8, bei dem die besagte Platte die Form eines gleichseitigen Dreiecks hat.

10. Werkzeug gemäß einem der Ansprüche 3 bis 9, **dadurch gekennzeichnet, dass** der besagte erste Punkt (P1) durch den Körper der besagten Platte hindurch verläuft.

11. Werkzeug gemäß Anspruch 8 und 10, **dadurch gekennzeichnet, dass** der besagte erste Punkte im Mittelpunkt oder in dichter Nähe des Mittelpunkts des Dreiecks liegt, wenn die Platte dreieckig ist.

12. Werkzeug gemäß einem der Ansprüche 3-11, **dadurch gekennzeichnet, dass** der besagte Tragarm (6) eine feste Länge hat.

13. Werkzeug gemäß einem der Ansprüche 3 bis 11, **dadurch gekennzeichnet, dass** der besagte Tragarm (6) eine veränderbare Länge hat.

## Revendications

1. Procédé permettant de stabiliser le sol par la construction d'une paroi moulée dont l'extrémité inférieure est ancrée dans une cavité sous alésée remplie de béton, le procédé consistant à construire une tranchée (10) puis à sous aléser une cavité (2) à l'intérieur de celle-ci en abaissant dans la tranchée un outil ayant un moyen d'excavation opérable pour engager la terre devant être excavée et continuellement en rotation pour excaver le sol ainsi engagé avec lui, **caractérisé en ce que** ladite rotation d'excavation continue est effectuée autour d'un axe généralement parallèle au fond de la tranchée.

2. Outil permettant de sous aléser une cavité dans une tranchée pour une paroi moulée construite selon le procédé de la revendication 1, **caractérisé en ce que** l'outil comporte un outil de coupe (8) continuellement en rotation autour d'un axe généralement parallèle au fond de la tranchée pour excaver le sol ainsi engagé avec lui dans le sens dudit axe et monté sur un bras (6) susceptible d'être oscillé de manière pivotante autour dudit axe en vue d'agrandir la zone excavée selon une largeur supérieure à la largeur de la tranchée.

3. Outil selon la revendication 2, **caractérisé en ce que** l'outil comporte un bras de positionnement (5) ; un bras de support (6) attaché de manière pivotante sur un premier point (P1) qui est au niveau ou à proximité d'une extrémité dudit bras de positionnement ; un outil de coupe (8) porté par ledit bras de support et monté de manière pivotante sur celui-ci sur un deuxième point (P2) qui est au niveau ou à proximité de cette extrémité du bras de support à distance dudit bras de positionnement ; une plaque (7) fixée en position par rapport audit bras de support ou de manière intégrale avec celui-ci, la plaque reposant dans le plan, ou parallèle au plan, balayé par ledit bras de support alors qu'il tourne autour dudit premier point ; et un moyen (9) destiné à faire bouger ladite plaque de telle manière à mener le bras de support à pivoter autour dudit premier point, permettant de ce fait un mouvement contrôlé dudit outil de coupe circulaire selon une trajectoire arquée.

4. Outil selon la revendication 3, **caractérisé en ce que** ledit bras de positionnement est un organe allongé.

5. Outil selon la revendication 3 ou la revendication 4, **caractérisé en ce que** ledit outil de coupe est circulaire en termes de forme.

6. Outil selon la revendication 3, la revendication 4, ou la revendication 5, **caractérisé en ce que** ledit moyen (9) destiné à faire bouger ladite plaque de telle manière à mener le bras de support à pivoter autour dudit premier point est une tringlerie rigide ou semi-rigide (12) se prolongeant entre (a) un point de connexion sur la plaque elle-même et (b) une position à proximité de l'axe du bras de positionnement et sur ce côté dudit premier point en face dudit deuxième point de pivotement quand le bras de support est positionné de telle manière à se prolonger en tant que prolongement du bras de positionnement.

7. Outil selon la revendication 6, **caractérisé en ce que** ladite tringlerie est mise en oeuvre par un vérin hydraulique.

8. Outil selon l'une quelconque des revendications 3 à 7, **caractérisé en ce que** ladite plaque est triangulaire.

9. Outil selon la revendication 8, dans lequel ladite plaque est sous la forme d'un triangle équilatéral.

10. Outil selon l'une quelconque des revendications 3 à 9, **caractérisé en ce que** ledit premier point (P1) passe au travers du corps de ladite plaque.

11. Outil selon la revendication 8 et la revendication 10, **caractérisé en ce que** ledit premier point est au niveau ou à proximité du centroïde du triangle quand la plaque est triangulaire.

12. Outil selon l'une quelconque des revendications 3 à 11, **caractérisé en ce que** ledit bras de support (6) est fixe en termes de longueur.

13. Outil selon l'une quelconque des revendications 3 à 11, **caractérisé en ce que** ledit bras de support (6) est variable en termes de longueur.
